# EUROPÄISCHE PATENTSCHRIFT

(11) **EP 2 446 253 B1**
(45) Veröffentlichungstag und Bekanntmachung des Hinweises auf die Patenterteilung: **12.02.2014**
(21) Anmeldenummer: 10747791.1
(22) Anmeldetag: 22.06.2010
(51) Int. Cl.: G01N 25/48, G01N 21/62, G01N 15/08, G01N 33/20, B01D 53/26

(54) **VERFAHREN UND VORRICHTUNG ZUR BESTIMMUNG DER ADSORPTION EINES GASES AN WERKSTOFFEN**
METHOD AND DEVICE FOR DETERMINING THE ADSORPTION OF A GAS ON MATERIALS
PROCÉDÉ ET DISPOSITIF POUR DÉTERMINER L'ADSORPTION D'UN GAZ SUR DES MATÉRIAUX

(30) Priorität: 26.06.2009 DE 102009031764
(43) Veröffentlichungstag der Anmeldung: 02.05.2012
(73) Patentinhaber: Fraunhofer-Gesellschaft zur Förderung der angewandten Forschung e.V., 80686 München (DE)
(72) Erfinder: KASKEL, Stefan, 01159 Dresden (DE); WOLLMANN, Phillip, 01069 Dresden (DE); LEISTNER, Matthias, 01187 Dresden (DE)
(74) Vertreter: Pfenning, Meinig & Partner GbR
(86) Internationale Anmeldenummer: PCT/DE2010/000756
(87) Internationale Veröffentlichungsnummer: WO 2010/149152

(56) Entgegenhaltungen:
- EP-A1- 1 775 015
- EP-A2- 0 260 942
- DE-A1- 4 431 932
- DE-A1- 10 019 122
- US-A1- 2005 082 482

## Beschreibung

Die Erfindung betrifft ein Verfahren und eine Vorrichtung zur Bestimmung der Adsorption eines Gases an Werkstoffen. Dabei kann auf die Werkstoffeigenschaften, wie Oberfläche, Porosität oder Affinität zu Gasen geschlossen werden. Es können auch Aussagen über das Adsorptionsverhalten von Werkstoffen zu Gasen oder Gasgemischen gewonnen werden, was beispielsweise für die Trennung einzelner Gase aus Gasgemischen von Bedeutung sein kann.

Häufig interessieren bei neu entwickelten oder in neuer Form hergestellten Werkstoffen, die in Rede stehenden Eigenschaften. Es bereitet aber Probleme und ist mit hohem Aufwand verbunden, entsprechende Untersuchungen durchzuführen, um beispielsweise die Affinität von Werkstoffen zu Gasen bestimmen zu können.

In der Vergangenheit hat man kalorimetrisch die Adsorptionswärme bestimmt und es wurden auch gravimetrische Messungen durchgeführt. Letztgenannte erfordern aber hoch genaue Messtechnik, da die Veränderung der Masse durch die Adsorption eines Gases an der Werkstoffoberfläche äußerst gering ist.

Kalorimetrisch wurde die Adsorptionswärme mit Tian-Calvet-Kalorimetern oder einem in DE 100 19 122 A1 beschriebenen Verfahren und Messaufbau bestimmt. Hierbei sind, da die sich durch Adsorption auftretenden Temperaturänderungen relativ klein sind, ein erheblicher messtechnischer Aufwand, mit einer Vielzahl von Temperatursensoren und ein großer Aufwand für die Thermostatierung erforderlich. Auch der erforderliche Zeitaufwand ist hoch, da sich die Messungen zumindest über mehrere Stunden hinziehen. Es ist ein Mindestprobenvolumen erforderlich.

Dadurch sind diese bisher bekannten Lösungen für schnelle insbesondere vergleichende Untersuchungen, die an neu entwickelten Werkstoffen oder solche die mit neuen bzw. veränderten Herstellungsverfahren erhalten werden, nur bedingt geeignet.

Es ist daher Aufgabe der Erfindung, Möglichkeiten für die Bestimmung der Oberflächeneigenschaften von Werkstoffen vorzuschlagen, bei denen bei ausreichender Messgenauigkeit mit reduziertem anlagentechnischen und Zeitaufwand Aussagen erhalten werden, und sehr kleine Probenvolumina untersucht werden können.

Erfindungsgemäß wird diese Aufgabe mit einem Verfahren gemäß Anspruch 1 gelöst. Die Bestimmung kann mit einer Vorrichtung, die die Merkmale des Anspruchs 15 aufweist, durchgeführt werden. Vorteilhafte Ausgestaltungen und Weiterbildungen der Erfindung sind bei Einsatz von in untergeordneten Ansprüchen bezeichneten Merkmalen erreichbar.

Bei der Erfindung wird eine Probe eines Werkstoffs innerhalb einer Kammer angeordnet. Die Kammer ist für elektromagnetische Strahlung im Wellenlängenbereich zwischen 150 nm bis 25 µm nicht transparent. Zur Bestimmung der Adsorption eines Gases am Probenwerkstoff wird ein Gas oder Gasgemisch der Kammer zugeführt und die Probe damit beaufschlagt, so dass das Gas oder zumindest ein in einem Gasgemisch enthaltendes Gas an der Oberfläche der Probe adsorbiert. Da bei der Adsorption eine Erwärmung auftritt, kann diese Temperaturerhöhung gemessen werden. Hierfür ist in der Kammer mindestens ein optischer Detektor angeordnet. Der Detektor ist zumindest in einem Bereich des Wellenlängenbereichs zwischen 150 nm und 25 µm sensitiv. Bevorzugt kommen Infrarot-Sensoren zum Einsatz. Mit dem/den Detektor(en) kann die in Folge der Adsorption von der Probe emittierte elektromagnetische Strahlung detektiert werden. Die erfassten Messsignale des/der Detektors/Detektoren werden dabei zeitaufgelöst erfasst und innerhalb eines vorgebbaren Zeitintervalls zur Bestimmung der durch die Adsorption sich ändernden Oberflächentemperatur und/oder der Adsorptionswärme der jeweiligen Probe ausgewertet.

Das jeweilige adsorbierende Gas oder Gasgemisch sollte über eine vorgebbare Zeit mit gesteuertem und/oder geregeltem Partialdruck der Kammer und somit auch der Probe zugeführt werden. Dies kann durch gezielte Beeinflussung des Volumenstromes über eine Gaszufuhr in die Kammer erreicht werden.

Für die Auswertung oder auch einen Vergleich verschiedener Proben kann die in Folge der Adsorption aufgetretene maximale Temperatur und/oder durch eine Integration des erfassten Messsignals innerhalb eines vorgegebenen Zeitintervalls die jeweilige Adsorptionswärme genutzt werden.

Insbesondere für vergleichende Untersuchungen oder auch zur Erhöhung der Auswertegenauigkeit können mehrere Proben gleichzeitig in einer Kammer einem adsorbierenden Gas oder einem ein solches Gas enthaltendem Gasgemischstrom ausgesetzt werden. In diesem Fall ist jeder Probe mindestens ein Detektor zugeordnet.

Es können auch mehrere Detektoren einer Probe zugeordnet werden. Dabei sollten die Detektoren so ausgerichtet oder angeordnet sein, dass unterschiedliche Oberflächenbereiche der Probe erfasst werden. Dies können ganz oder teilweise andere Oberflächenbereiche sein, wenn beispielsweise aus anderen Richtungen detektiert wird. Es besteht aber auch die Möglichkeit mit einem Detektor einen kleineren Oberflächenbereich und mit einem weiteren Detektor einen größeren Oberflächenbereich, in dem der kleinere Oberflächenbereich enthalten ist, zu detektieren. Dabei können gleiche Detektoren in unterschiedlichen Abständen zur Probenoberfläche angeordnet oder eine Einstellung einer bestimmten Brennweite ermöglichende Optik zumindest an einem Detektor vorhanden sein.

Die eingesetzten Detektoren können so ausgewählt oder ausgebildet sein, dass sie für bestimmte Wellenlängen oder Wellenlängenbereiche sensitiv sind und diese dann nur bei der Messung berücksichtigt werden. Hierfür können geeignete optische Filter, beispielsweise Bandpass- oder Kantenfilter zwischen Detektor und Probe angeordnet werden. Es können auch durchstimmbare Detektoren (Infrarot-Fabry-Perot-Interferometer) eingesetzt werden. Diese können auf eine Wellenlänge bzw. ein eng begrenztes Wellenlängenintervall abgestimmt sein, das für die Messung besonders geeignet ist. Dadurch kann das Messsignal einen kleineren Anteil an Störgrößen aufweisen.

Bei der Bestimmung kann so vorgegangen werden, dass zuerst, also bevor die eigentliche Bestimmung durchgeführt wird, ein nicht oder nur geringfügig adsorbierendes Gas kontinuierlich durch die Kammer hindurchgeführt wird. Nach einer vorgebbaren Zeit wird dann diesem Gas ein an der Probe adsorbierendes Gas mit konstantem Partialdruck über ein vorgegebenes Zeitintervall zugemischt. Die durch die Adsorption auftretende Temperaturerhöhung führt dazu, dass von der Probe elektromagnetische Strahlung emittiert wird, die mit dem mindestens einen Detektor erfasst und gemessen werden kann. In der Regel ist das Messsignal eine der Temperatur proportionale elektrische Spannung, die über die Zeit erfasst werden kann.

Als nicht oder nur geringfügig adsorbierendes Gas können Stickstoff, Helium oder Wasserstoff und als adsorbierendes Gas Methan, Alkane mit der allgemeinen Formel CₙH₂ₙ₊₂ wie z.B. Ethan, Propan, CO, CO₂, H₂O, NOₓ, NH₃, H₂S oder Butan eingesetzt werden. Weitere absorbierende Gase können Alkene, Alkine, Polyene sein. An deren Stelle können aber auch Lösungsmitteldämpfe von Aromaten, wie Benzol, Toluol, Ketone (Aceton), Ester oder Thiole oder auch Alkohol(-dämpfe) untersucht werden.

Das zugeführte Gas oder Gasgemisch sollte temperiert werden, bevor es in die Kammer für die Bestimmung zugeführt wird. Im Gegensatz zu den bekannten technischen Lösungen kann bei normalen Umgebungstemperaturen verfahren, also bei Raumtemperatur gearbeitet werden. Es kann aber auch auf Temperaturen bis zu 120°C, bevorzugt bis zu 100°C und auch auf eine Temperatur von ca. 77 K temperiert werden. Bei dieser Temperatur können Stickstoff, Argon und Wasserstoff als adsorbierendes Gas und dabei Helium als nicht oder nur geringfügig adsorbierendes Gas eingesetzt werden. Die Temperatur sollte lediglich konstant gehalten werden. Hierfür kann an einer Gaszufuhr zur Kammer eine regelbare Heizeinrichtung vorhanden sein.

Es besteht die Möglichkeit, im Anschluss an eine durchgeführte Adsorption die Zufuhr von Gas oder Gasgemisch zu beenden und dann bei gleichzeitiger Energiezufuhr das freigesetzte vorab adsorbierte Gasvolumen zu bestimmen. Die Energiezufuhr kann mittels einer in die Kammer integrierten Heizeinrichtung erfolgen. Dabei kann eine Probe an einer solchen Heizeinrichtung angeordnet sein oder die Energiezufuhr durch Bestrahlung mit elektromagnetischer Strahlung erreicht werden.

Vorteilhaft sollten vorab aktivierte Proben in die Kammer eingesetzt werden. Eine Aktivierung der Proben kann auch innerhalb der Kammer durch Zufuhr eines auf eine Temperatur oberhalb 50°C, bevorzugt bei 70°C erwärmten nicht oder nur geringfügig adsorbierenden Gases erreicht werden.

Mit der Erfindung können die in Rede stehenden Untersuchungen innerhalb verhältnismäßig kurzer Zeit, die 20 min nicht überschreitet und in der Regel kürzer ist, durchgeführt werden. Wie bereits angesprochen können gleichzeitig mehrere Proben berücksichtigt und die Bestimmung gemeinsam durchgeführt werden. Es können sehr kleine Proben untersucht werden. Es können Proben mit einer Eigenmasse von wenigen mg (ca. 2 bis 20 mg) untersucht werden. Dabei sollte lediglich beachtet werden, dass auf einem Detektor möglichst ausschließlich von einer Probe emittierte elektromagnetische Strahlung abgebildet und diese gemessen wird.

Durch die erreichten Vorteile kann die Erfindung insbesondere für Schnelltests eingesetzt werden, bei denen die prinzipielle Eignung neuer Werkstoffe festgestellt oder mit einem neuen oder zu optimierenden Herstellungsverfahren hergestellte Werkstoffe untersucht und verbessert werden können. Die Untersuchungen müssen nicht bei stark reduzierten oder erhöhten Drücken und Temperaturen durchgeführt werden. Der Aufwand für die thermische Isolation ist gering, da die erforderliche Messzeit im Vergleich zum Stand der Technik klein gehalten werden kann. Es sollte lediglich darauf geachtet werden, dass innerhalb der Kammer keine oder nur geringfügig störende elektromagnetische Strahlung auftritt, die nicht in Folge der Adsorption auftritt. Für den Fall, dass störende elektromagnetische Strahlung nicht vollständig vermieden werden kann, besteht die Möglichkeit diese durch optische Filter bei der Messung auszublenden oder durch eine Einstellung eines durchstimmbaren Detektors vorzunehmen, so dass dieser bei den störenden Wellenlängen nicht sensitiv ist.

Nachfolgend soll die Erfindung beispielhaft näher erläutert werden.

Dabei zeigen:
Figur 1 in schematischer Form den Aufbau einer erfindungsgemäßen Vorrichtung und
Figur 2 ein Diagramm mit dem vergleichend Untersuchungen an unterschiedlichen Proben verdeutlicht sind.

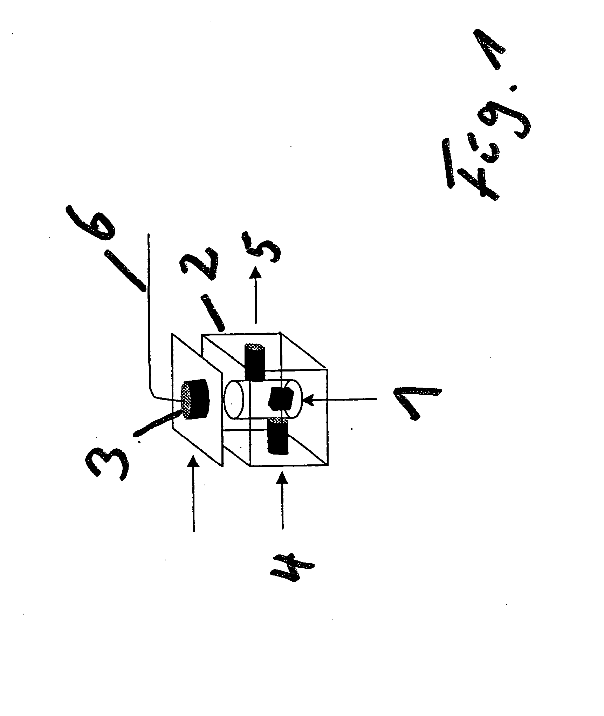

In Figur 1 ist schematisch der prinzipielle Aufbau einer für erfindungsgemäß durchzuführende Untersuchungen geeigneten Vorrichtung dargestellt.

Bei diesem Beispiel sind in einer allseitig, bis auf eine Gaszufuhr 4 und einen Gasauslass 5, abgeschlossenen Kammer 2 lediglich eine Probe 1 und ein optischer Detektor 3, der hier ein Infrarot-Sensor mit einer Sensitivität im Wellenlängenbereich von 5 µm bis 20 µm ist, angeordnet. Die Kammer 2 ist aus einem nicht transparenten Werkstoff gebildet.

Der Detektor 3 ist über eine Messsignalleitung 6 an eine hier nicht dargestellte elektronische Auswerteeinheit angeschlossen.

Nach dem Einsetzen einer Probe 1 in die Kammer 2 und deren Verschluss, erfolgte eine Aktivierung des Probenwerkstoffs durch die Zufuhr von Stickstoff über die Gaszufuhr 4, der auf eine Temperatur von 70°C erwärmt worden ist. Die Aktivierung wurde über eine Zeit von 30 min durchgeführt.

Anschließend daran wurde über eine Zeit von 30 s reiner Stickstoff bei konstant gehaltener Temperatur von 23°C in die Kammer mit einem Volumenstrom von 30 ml/min eingeführt und dann wieder über den Gasauslass 5 abgeführt. Die Kammer 2 hatte ein freies Innenvolumen von 0,5 cm³.

Nach Ablauf dieser Zeit wurden dem Stickstoff Butan mit einer Dosiereinrichtung vor dem Eintritt in die Kammer 2 zugemischt. Es wurde ein Volumenstrom von 30 ml/min für Stickstoff und 10 ml/min für Butan eingestellt. Es sind aber auch für die Untersuchungen andere Verhältnisse und Volumenströme nutzbar.

Es wurden vergleichende Untersuchungen mit Proben 1 aus fünf unterschiedlichen Werkstoffen durchgeführt.

Dabei handelte es sich um die folgenden Werkstoffe:
MIL-100_{Fe} Fe₃F(H₂O)₃O[C₅H₃-(CO₂)₃]₂nH₂O
MIL-101 Cr₃F(H₂O)₂O[(O₂C)-(C₆ H₄)-(CO₂)]₃nH₂O
Cu-BTC Cu₃(C₅H₃(CO₂)₃)₂(H₂O)₃
Zn-NDC-dabco Zn₂[C₁₀H₆(CO₂)₂]₂-C₅H₁₂N₂
und
Aktivkohle.

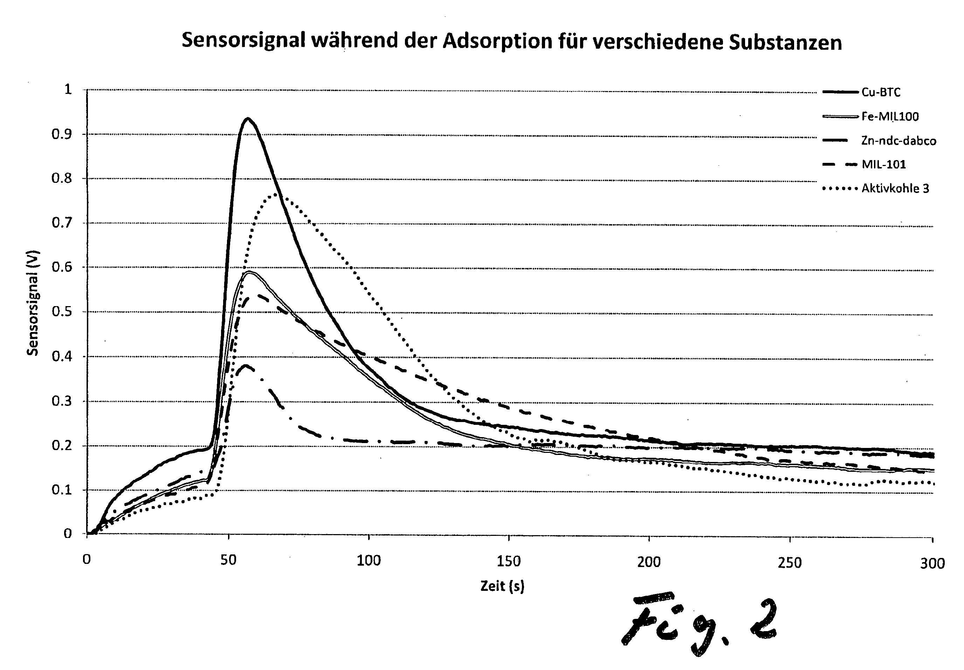

Die mit dem Detektor 3 erfassten Messsignalverläufe über die Zeit sind für die fünf Werkstoffe im Diagramm der Figur 2 gezeigt. Die Masse der fünf Proben betrug jeweils 20 mg.

Es zeigte sich, dass nach kurzer Zeit ab ca. 50 s das jeweilige Maximum erreicht worden ist und anschließend eine Abklingphase in Folge der Sättigung auftrat.

Die Messsignalverläufe entsprechen den jeweiligen Temperaturen an der Probenoberfläche, die sich durch die Adsorption des Butans an der Probenoberfläche entsprechend erhöhte.

Für die Auswertung der einzelnen Proben 1 konnten die kleinsten Messfehler bei der Auswertung des jeweiligen maximalen Messsignals und dessen Integral über die Zeit bis zum Erreichen des Ausgangswertes des Messsignals vor Beginn der Zumischung von Butan, nachgewiesen werden. Hierzu wurden Vergleichsuntersuchungen mit einer Butanwaage durchgeführt. Das Integral ist proportional zur jeweiligen Adsorptionswärme bzw. der Kapazität des Adsorbens gegenüber Butan.

Für die Auswertung können aber auch der Anstieg bis zum Erreichen des Maximalwerts, die Zeit bis zum Erreichen des Ausgangswertes oder der Anstieg des Messsignalverlaufs ausgehend vom Maximum bis zum Erreichen des Ausgangswertes herangezogen werden.

Im Anschluss an diese Adsorptionsuntersuchungen wurde eine Desorption der Proben 1 durchgeführt. Dabei wurde zuerst auf 70°C erwärmter reiner Stickstoff in die Kammer 2 hinein und wieder heraus geführt. Dabei wurden 60 ml/min über eine Zeit von 30 min eingesetzt.

## Patentansprüche

1. Verfahren zur Bestimmung der Adsorption eines Gases an Werkstoffen, bei dem eine Probe (1) eines Werkstoffs innerhalb einer Kammer (2), die für elektromagnetische Strahlung im Wellenlängenbereich zwischen 150 nm bis 25 µm nicht transparent ist, mit einem Gas oder Gasgemisch beaufschlagt wird, das oder zumindest ein in einem Gasgemisch enthaltendes Gas an der Oberfläche der Probe (1) adsorbiert, dabei mit mindestens einem optischen Detektor (3), der zumindest in einem Bereich des Wellenlängenbereichs zwischen 150 nm und 25 µm sensitiv ist, die in Folge der Adsorption von der Probe (1) emittierte elektromagnetische Strahlung detektiert und die Messsignale des/der Detektors/Detektoren (3) zeitaufgelöst erfasst und innerhalb eines vorgebbaren Zeitintervalls zur Bestimmung der durch die Adsorption sich ändernden Oberflächentemperatur und/oder der Adsorptionswärme der jeweiligen Probe (1) ausgewertet werden.

2. Verfahren nach Anspruch 1, **dadurch gekennzeichnet, dass** Gas oder Gasgemisch über eine vorgebbare Zeit mit gesteuertem und/oder geregeltem Partialdruck der Kammer (2) zugeführt wird.

3. Verfahren nach Anspruch 1 oder 2, **dadurch gekennzeichnet, dass** die in Folge der Adsorption erreichte maximale Temperatur bestimmt wird.

4. Verfahren nach einem der vorhergehenden Ansprüche, **dadurch gekennzeichnet, dass** ein oder mehrere Infrarot-Sensoren eingesetzt werden.

5. Verfahren nach einem der vorhergehenden Ansprüche, **dadurch gekennzeichnet, dass** mit dem/den Messsignalen durch Integration innerhalb des vorgegebenen Zeitintervalls die Adsorptionswärme bestimmt wird.

6. Verfahren nach einem der vorhergehenden Ansprüche, **dadurch gekennzeichnet, dass** mit optischen Detektoren (3) unterschiedliche Oberflächenbereiche der Probe (1) erfasst werden.

7. Verfahren nach einem der vorhergehenden Ansprüche, **dadurch gekennzeichnet, dass** ein nicht oder nur geringfügig adsorbierendes Gas kontinuierlich durch die Kammer (2) hindurchgeführt wird und für die Bestimmung der Adsorption diesem Gas ein an der Probe adsorbierendes Gas mit konstantem Partialdruck über ein vorgegebenes Zeitintervall zugemischt wird.

8. Verfahren nach Anspruch 6, **dadurch gekennzeichnet, dass** als nicht oder nur geringfügig adsorbierendes Gas Stickstoff, Helium oder Wasserstoff und als adsorbierendes Gas Alkoholdampf, Methan, Ethan, Propan, CO, CO₂, H₂O, NOₓ, NH₃, H₂S oder Butan eingesetzt werden.

9. Verfahren nach einem der vorhergehenden Ansprüche, **dadurch gekennzeichnet, dass** zugeführtes Gas und Gasgemisch auf normale Umgebungstemperatur oder im Bereich Umgebungstemperatur bis zu 120 °C temperiert werden.

10. Verfahren nach einem der vorhergehenden Ansprüche, **dadurch gekennzeichnet, dass** mehrere Proben (1) innerhalb der Kammer (2) gleichzeitig detektiert und miteinander verglichen werden.

11. Verfahren nach einem der vorhergehenden Ansprüche, **dadurch gekennzeichnet, dass** im Anschluss an eine durchgeführte Adsorption die Zufuhr von Gas oder Gasgemisch beendet und bei gleichzeitiger Energiezufuhr das freigesetzte vorab adsorbierte Gasvolumen bestimmt wird.

12. Verfahren nach einem der vorhergehenden Ansprüche, **dadurch gekennzeichnet, dass** vorab aktivierte Proben (1) in die Kammer (2) eingesetzt oder Proben (1) innerhalb der Kammer (2) durch Zufuhr eines auf eine Temperatur oberhalb 50°C erwärmten nicht oder nur geringfügig adsorbierenden Gases aktiviert werden.

13. Vorrichtung zur Bestimmung der Adsorption eines Gases an Werkstoffen, bei der an einer Kammer (2), die für elektromagnetische Strahlung im Wellenlängenbereich zwischen 150 nm bis 25 µm nicht transparent ist, eine Gaszufuhr (4) und ein Gasauslass (5) vorhanden sind, und auf eine innerhalb der Kammer (2) angeordnete Probe (1) eines Werkstoffs mindestens ein für elektromagnetische Strahlung im Wellenlängenbereich zwischen 150 nm und 25 µm sensitiver optischer Detektor (3) gerichtet und an eine elektronische Auswerteeinheit angeschlossen ist.

14. Vorrichtung nach Anspruch 13, **dadurch gekennzeichnet, dass** mindestens zwei optische Detektoren (3) in unterschiedlichen Winkeln auf die Probe (1) gerichtet sind und/oder durch optische Elemente unterschiedlich große Oberflächenbereiche einer Probe (1) auf den optischen Detektoren (3) abgebildet werden.

15. Vorrichtung nach Anspruch 13 oder 14, **dadurch gekennzeichnet, dass** mit mehreren optischen Detektoren (3) unterschiedliche Wellenlängen mit eingesetzten optischen Filtern und/oder mit mindestens einem durchstimmbaren optischen Detektor (3) erfassbar sind.

## Claims

1. Method for determining the adsorption of a gas at materials, wherein a sample (1) of a material within a chamber (2) which is not transparent to electromagnetic radiation in the wavelength range between 150 nm and 25 µm is acted on by a gas or gas mixture, which gas or at least a gas included in a gas mixture is adsorbed at the surface of the sample (1), the electromagnetic radiation emitted by the sample (1) as a consequence of the adsorption being detected by at least one optical detector (3) which is sensitive at least in a range of the wavelength range between 150 nm and 25 µm, and the measured signals of the detector(s) (3) being detected with time resolution and evaluated within a predefinable time interval for determining the surface temperature and/or the adsorption heat of the respective sample (1) varying due to the adsorption.

2. Method according to Claim 1, **characterised in that** the gas or gas mixture is supplied to the chamber (2) over a predefinable time with a controlled and/or regulated partial pressure.

3. Method according to Claim 1 or 2, **characterised in that** the maximum temperature achieved as a consequence of the adsorption is determined.

4. Method according to one of the preceding claims, **characterised in that** one or more infrared sensors are used.

5. Method according to one of the preceding claims, **characterised in that** the adsorption heat is determined using the measured signal(s) by integration within the predefined time interval.

6. Method according to one of the preceding claims, **characterised in that** different surface regions of the sample (1) are detected using optical detectors (3).

7. Method according to one of the preceding claims, **characterised in that** a non-adsorbing gas or an only slightly adsorbing gas is continuously conducted through the chamber (2) and a gas adsorbing at the sample is admixed to this gas at a constant partial pressure over a predefined time interval for determining the adsorption.

8. Method according to Claim 6, **characterised in that** nitrogen, helium or hydrogen are used as the non-adsorbing gas or only slightly adsorbing gas and alcohol vapour, methane, ethane, propane, CO, CO₂, H₂O, NOₓ, NH₃, H₂S or butane are used as the adsorbing gas.

9. Method according to one of the preceding claims, **characterised in that** the supplied gas and gas mixture are temperature-controlled to normal ambient temperature or to a temperature in the range of ambient temperature up to 120°C.

10. Method according to one of the preceding claims, **characterised in that** a plurality of samples (1) are simultaneously detected within the chamber (2) and are compared with one another.

11. Method according to one of the preceding claims, **characterised in that** subsequent to an adsorption carried out, the supply of gas or of gas mixture is ended and the released previously adsorbed gas volume is determined with a simultaneous energy supply.

12. Method according to one of the preceding claims, **characterised in that** previously activated samples (1) are introduced into the chamber (2) or samples (1) are activated within the chamber (2) by supply of a non-adsorbing gas or an only slightly adsorbing gas heated to a temperature above 50°C.

13. Apparatus for determining the adsorption of a gas at materials, wherein a gas supply (4) and a gas outlet (5) are present at a chamber (2) which is not transparent to electromagnetic radiation in the wavelength range between 150 nm and 25 µm and at least one optical detector (3) sensitive to electromagnetic radiation in the wavelength range between 150 nm and 25 µm is directed towards a sample (1) of a material arranged within the chamber (2) and is connected to an electronic evaluation unit.

14. Apparatus according to Claim 13, **characterised in that** at least two optical detectors (3) are directed towards the sample (1) at different angles and/or different-size surface regions of a sample (1) are imaged on the optical detectors (3) by optical elements.

15. Apparatus according to Claim 13 or 14, **characterised in that** different wavelengths can be detected using a plurality of optical detectors (3) with inserted optical filters and/or with at least one adjustable optical detector (3).

## Revendications

1. Procédé de détermination de l'adsorption d'un gaz sur des matériaux, dans lequel, à l'intérieur d'une chambre (2) qui n'est pas transparente pour le rayonnement électromagnétique dans l'intervalle de longueur d'ondes allant de 150 nm à 25 µm, un échantillon (1) d'un matériau est soumis à un gaz ou à un mélange de gaz, le gaz ou au moins un gaz contenu dans un mélange de gaz s'adsorbant à la surface de l'échantillon (1), dans lequel au moins un détecteur optique (3) qui est sensible dans au moins un intervalle à l'intérieur de l'intervalle de longueurs d'ondes allant de 150 nm à 25 µm, détecte le rayonnement électromagnétique émis par l'échantillon à la suite de l'adsorption et enregistre les signaux de mesure du(des) détecteur(s) (3) par résolution temporelle et, à l'intérieur d'un intervalle de temps qui peut être prédéfini, les évalue pour la détermination de la température superficielle qui varie en raison de l'adsorption, et/ou de la chaleur d'adsorption de l'échantillon (1) respectif.

2. Procédé selon la revendication 1, **caractérisé en ce que** le gaz ou le mélange de gaz est acheminé dans la chambre (2) sur une période prédéfinie sous une pression partielle commandée et/ou régulée.

3. Procédé selon la revendication 1 ou 2, **caractérisé en ce que** la température maximale atteinte à la suite de l'adsorption est déterminée.

4. Procédé selon l'une quelconque des revendications précédentes, **caractérisé en ce qu'**un ou plusieurs capteurs d'infrarouge sont utilisés.

5. Procédé selon l'une quelconque des revendications précédentes, **caractérisé en ce que** la chaleur d'adsorption est déterminée avec le(les) signal(aux) de mesure par intégration à l'intérieur de l'intervalle de temps prédéfini.

6. Procédé selon l'une quelconque des revendications précédentes, **caractérisé en ce que** différentes zones superficielles de l'échantillon (1) sont mesurées avec des détecteurs optiques (3).

7. Procédé selon l'une quelconque des revendications précédentes, **caractérisé en ce qu'**un gaz ne s'adsorbant pas ou que légèrement traverse continuellement la chambre (2), et **en ce que**, pour la détermination de l'adsorption, un gaz s'adsorbant sur l'échantillon est mélangé à ce gaz sous une pression partielle constante pendant un intervalle de temps prédéfini.

8. Procédé selon la revendication 6, **caractérisé en ce que** comme gaz ne s'adsorbant pas ou ne s'adsorbant que légèrement sont utilisés de l'azote, de l'hélium ou de l'hydrogène et comme gaz adsorbant sont utilisés de la vapeur d'alcool, du méthane, de l'éthane, du propane, du CO, du CO₂, de l'H₂O, du NOₓ, du NH₃, du H₂S ou du butane.

9. Procédé selon l'une quelconque des revendications précédentes, **caractérisé en ce que** le gaz et le mélange de gaz acheminé est amené à la température ambiante normale ou dans un intervalle allant de la température ambiante à 120 °C.

10. Procédé selon l'une quelconque des revendications précédentes, **caractérisé en ce que** plusieurs échantillons (1) sont détectés simultanément à l'intérieur de la chambre (2) et comparés.

11. Procédé selon l'une quelconque des revendications précédentes, **caractérisé en ce qu'**à la suite de la réalisation d'une adsorption, l'alimentation en gaz ou en mélange de gaz est arrêtée et, lors de l'apport simultané en énergie, le volume de gaz dégagé, précédemment adsorbé, est déterminé.

12. Procédé selon l'une quelconque des revendications précédentes, **caractérisé en ce que** les échantillons préalablement activés (1) sont placés dans la chambre (2) ou **en ce que** les échantillons (1) sont activés à l'intérieur de la chambre (2) par l'alimentation en gaz ne s'adsorbant pas ou ne s'adsorbant que légèrement, réchauffé à une température supérieure à 50 °C.

13. Dispositif de détermination de l'adsorption d'un gaz sur des matériaux, pour lequel une chambre (2) qui n'est pas transparente au rayonnement électromagnétique dans l'intervalle de longueur d'onde allant de 150 nm à 25 µm, est dotée d'une arrivée de gaz (4) et d'une sortie de gaz (5), et pour lequel au moins un détecteur optique (3) sensible au rayonnement électromagnétique dans l'intervalle de longueur d'onde allant de 150 nm à 25 µm est dirigé sur un échantillon (1) d'un matériau disposé à l'intérieur de la chambre (2), et est raccordé à une unité d'évaluation électronique.

14. Dispositif selon la revendication 13, **caractérisé en ce qu'**au moins deux détecteurs optiques (3) sont dirigés sur l'échantillon (1) sous des angles différents et/ou **en ce que** des zones superficielles de différentes tailles d'un échantillon (1) sont reproduites sur les détecteurs optiques (3) par des éléments optiques.

15. Dispositif selon la revendication 13 ou 14, **caractérisé en ce qu'**avec plusieurs détecteurs optiques (3), différentes longueurs d'onde peuvent être mesurées par des filtres optiques intercalés et/ou par au moins un détecteur optique (3) ajustable.
